# EUROPÄISCHE PATENTANMELDUNG

(11) **EP 3 575 417 A1**
(43) Veröffentlichungstag der Anmeldung: **04.12.2019**
(21) Anmeldenummer: 18174983.9
(22) Anmeldetag: 30.05.2018
(51) Int. Cl.: C12Q 1/6895

(54) **VERWENDUNG VON CMS MARKER ZUR ZYTOPLASMABESTIMMUNG**

(71) Anmelder: Strube Research GmbH & Co. KG, 38387 Söllingen (DE)
(72) Erfinder: Dr. Benke, Andreas, D-38387 Söllingen (DE)
(74) Vertreter: Helbig, Christian

(57) **Zusammenfassung**

Die Erfindung betrifft QTL und CMS Marker, Verfahren zum Screenen und/oder zur Selektion sowie die Verwendung der Marker hierzu.

SEQUENCE LISTING

<110> Strube Research GmbH & Co. KG

<120> Verwendung von CMS Marker zur Zytoplasma Bestimmung

<130>

<160> 11

<170> PatentIn version 3.5

<210> 1
<211> 101
<212> DNA
<213> Triticum aestivum

<400> 1
cgacgacgac gaggatgccg agtttgatga catggaggat tatatcgacg rcgcggactg 60

ggacgccgac atgtatgatg atgtgttcga tgtctgaagg a 101

<210> 2
<211> 201
<212> DNA
<213> Triticum aestivum

<400> 2
tagttttctt cttggttgcc tgtaataaca tcataggttg aggttaccag ccgctccgca 60

taaagcttga aagttgaact tgctgaagcg gcgagcctcg ygagcaccac caccaccgac 120

aagtatcagg agcacctttc cctccagtac atgtaaataa aaaggagtat acatatttgt 180

cctatgtatc gatttgagaa t 201

<210> 3
<211> 101
<212> DNA
<213> Triticum aestivum

<400> 3
aactttatgc atgcgccatt gcagatcaat atctagtaga tcaatcagct kctctcgcct 60

aacaggatca actccatcaa ctccaaatat catgtgctca g 101

<210> 4
<211> 885
<212> DNA
<213> Triticum timopheevii

<400> 4
cactagcagg tttactgctt tctatttgca cttttgtatt aagtttcctt atatatacga 60

ttttttatta ttttctattt gtctattttt ctttttagtg cgttttattt cgattattct 120

tctcccaatt tgcaatcttt tcggagcctc cttcattatt actcttcctc cagagattca 180

ggatccccaa gctctagctc atttagcagg gctaaacttc tatctgagcc tttacgagca 240

ggatcctgga tgggttacgt tcattcagaa cgagcttaat cacaataccc ctctggagga 300

catacctgga cggcttaagc tcttcctaat ggaagaaaag ctgtctagta tgcgacaaga 360

tgtcattcag gaatttgtgg cgctttatca aagaataggg ccttatctac cgatcgagcc 420

ctacttggtc gatgaagcgc ttcgttccta tctggaccat attcacgcaa ctgattcttt 480

cactgttctc caagcgtctt atcaagatct gcgggagaat gagggaggat ctgttttctt 540

tagagatgct gtttcccaca accgggatct ccttgaggcg gaaagctccg caaggaggtg 600

cctggaagtg gaacagagga tccgatggga agaaatcccc aagagcaagg caagtctcga 660

aagagctgag cacgagcatg ctctcgactt gtttaagtcg gaggatctta gaagggaatt 720

agaaaaaaaa agagcggggt agctcagtaa ttctgattct tttctcttcc agcccccggg 780

cgggcccctc tttcttttct cttccagccc cccggcccct ctttgataag gaaagtttgc 840

atttctcaaa taaaaaatga caaatatggt tcgatggctc ttctc 885

<210> 5
<211> 90
<212> DNA
<213> Triticum timopheevii

<400> 5
tatgygacaa gatgtcattc aggaatttgt ggcgctttat caaagartag ggccttatct 60

accgatcgag ccctacttgg tcratgaagc 90

<210> 6
<211> 90
<212> DNA
<213> Triticum timopheevii

<400> 6
tatgygacaa gatgtcattc aggaatttgt ggcgctttat caaagartag sgccttatct 60

acygatcgag ccctacttgg tcratgaagc 90

<210> 7
<211> 831
<212> DNA
<213> Triticum aestivum

<400> 7
cactacccgc tttactactt tctatttttt cttatgtatt aagtttccct taatatataa 60

tttgatatta ctttcagttt gtatattttt attcctttgt cgttttatat tatgcctaat 120

accaattttc aatcttttta gttcgtgctt ctcggctcct cctttctgat cactctccag 180

ccggagattc aagaccccta ggctctagct catttagcag ggctaaactt ctatctgagt 240

ctttacgagc aggatccgag atgggttaca ttcattcaga acgagcttaa tcacaatacc 300

ccccagaaga catacctggg cggcttaagc tcttcctaat ggaagaaaag ctctcttcta 360

tgcgacaaga tgtcattcag gaatttgtgg cgctttatca aagagtagcg ccttatctac 420

tgatcgagcc ctacttggtc gatgaagtgc ttcgttccta tctggaccat attcacgcaa 480

cttattcttt cactgttctc taagcgtctt atcaagatct gcgggagaat gagggaggat 540

ccgttttctt ttgaaatgtt gtttcccaca accgggatct ccttgaggcg gaaagctccg 600

caaggaggtg cctggaagtg gaacagagga tccggtggga agaaatcccc aagagcaagg 660

caagtcttga aagagctaag cacgagcatg ctctcgactt gtttaagtcg gagaatctta 720

gaagggaatt agaaaaaaaa gcagggtagc tcagtaattc tgattctttt ctcttccagc 780

cccctggccc ctctttcttt tctcttccag cccccgggcg ggcccctctt t 831

<210> 8
<211> 334
<212> DNA
<213> Triticum aestivum

<400> 8
gttacgttca ttcagaacga gcttaatcac aatacccctc tggaggacat acctggacgg 60

cttaagctct tcctaatgaa agaaaagctg tctagtatgt gacaagatgt cattcaggaa 120

tttgtggcgc tttatcaaag aatagggcct tatctaccga tcgagcccta cttggtcaat 180

gaagcgcttc gttcctatct ggaccatatt cacgcaactg attctttcac tgttctccaa 240

gcgtcttatc aagatctgcg ggagaataag ggaggatctg ttttctttag agatgttgtt 300

tcccacaacc gggatctcct tgaggcggaa agct 334

<210> 9
<211> 22
<212> DNA
<213> Triticum timopheevii

<400> 9
cgatcggtag ataaggccct at 22

<210> 10
<211> 22
<212> DNA
<213> Triticum aestivum

<400> 10
cgatcggtag ataaggccct ac 22

<210> 11
<211> 29
<212> DNA
<213> Triticum timopheevii & Triticum aestivum

<400> 11
cattcaggaa tttgtggcgc tttatcaaa 29

## Beschreibung

Die Erfindung betrifft QTL und CMS Marker, Verfahren zum Screenen und/oder zur Selektion sowie die Verwendung der Marker hierzu.

### Hintergrund der Erfindung

### Molekulare Marker und Pflanzenzüchtung

Molekulare Marker sind Nachweismittel, um bestimmte Eigenschaften auf molekularer Ebene zu identifizieren oder/und deren Auftreten zu verfolgen.

Eine Vielzahl von Markern kann zum Screenen oder Selektieren von Eigenschaften in Pflanzen oder Pflanzenzellen genutzt werden.

Hierbei stellen SNPs (single nucleotide polymorphism) eine besondere Gruppe von Markern dar, die auch als genetische Marker bezeichnet werden.

Die Verbindung von SNPs zu einem bestimmten Gen und damit einer bestimmten Eigenschaft (oder Phänotyp) kann mit bekannten genetischen Mitteln bestimmt werden. Diese Verbindung oder das Auftreten von einem bestimmten mit einem Marker in Verbindung stehenden Merkmal nennt man auch Marker-Phänotyp Assoziation.

In Züchtungsverfahren will man bestimmte Eigenschaften einer Pflanze anreichern, d.h. man selektiert auf bestimmte Merkmale oder Eigenschaften der Pflanze. Zum Nachweis von Merkmalen bzw. der Wahrscheinlichkeit des Auftretens eines Merkmals mittels Markern kann nun ein SNP oder eine Gruppe von SNPs verwendet werden.

Ein wichtiges Merkmal ist der Ertrag einer Pflanze. Hierbei hat sich herausgestellt, dass Hybrid-Sorten vorteilhafte Eigenschaften wie Ertragstabilität zeigen. In Hybrid-Sorten werden genetische Pools mit unterschiedlichen Eigenschaften, die auf männliche und weibliche Kreuzungspartner verteilt sein können, durch deren Kreuzung kombiniert.

Ein Ansatz auf dem Gebiet der Hybrid-Pflanzenherstellung ist die Verwendung von CMS (cytoplasmatic male sterility) Pflanzen. Es wurden erfolgreich HybridSorten in einer Reihe von Pflanzen wie Tabak hergestellt, die dieses System verwenden. Hierbei sind sog. Restorer-Gene (Rf) von Bedeutung.

Zum einen ist die Hybridzüchtung in Weizen kommerziell hauptsächlich von Gametoziden abhängig, die nicht nur phytotoxisch sein können, sondern der richtige Zeitpunkt der Applikation solcher entscheidend ist. Zum anderen muss das System kostengünstig in der Produktion sein. In der Vergangenheit wurden CMS Linien eingesetzt, die bestmöglich steril waren. Allerdings war die Fertilitätswiederherstellung durch die Restorer-Linien problematisch, sodass Verluste in der Einkörnung zu unbefriedigender Saatgutproduktionsmenge führten. Ein befriedigendes System in Bezug auf die Fertilitätswiederherstellung mittels Restorer-Linien konnte bisher nicht bereitgestellt werden. Auch wäre es wünschenswert, wenn die Fertilitätswiederherstellung mittels Restorer-Linien möglichst unabhängig von Umwelteinflüssen erzielt werden könnte.

Zwar erlauben phänotypische Analysen im Feld bisher die Detektion von fertilen Genotypen. Allerdings kann derselbe Genotyp im nächsten Jahr Fertilitätsprobleme aufweisen. Das führt dazu, dass eine große Zahl an potentiellen Restorer-Linien über mehrere Jahre angebaut und getestet werden muss, um vollständig Fertilität-restaurierende Restorer- Linien zu finden.

Somit ist eine Marker-gestützte Selektion wünschenswert, da diese die Detektion von Genotypen mit einem vollständigen Fertilitätsrestaurationssystem nicht nur beschleunigen, sondern dieses auch effizienter gestalten würde.

In Weizen war es bisher nicht möglich wirtschaftlich interessante Hybrid-Sorten mit dem Fertilitätsrestaurationssystem bereit zu stellen.

### Das CMS System

Zum CMS System (cytoplasmatic male sterility) wurden über viele Jahre große Anstrengungen unternommen, aber bisher konnten keine befriedigenden und praktikablen Marker oder Verfahren zum Screening im Hochdurchsatz bereitgestellt werden.

Molekulare Marker, die mit einer phänotypischen Eigenschaft korrelieren, werden zum Nachweis von qualitativen und quantitativen Merkmalen eingesetzt. Eine aktuell in der Praxis sehr etablierte bzw. bevorzugte Methode ist die Nutzung von SNPs (single nucleotide polymorphism) Markern, welche eine Punktmutation repräsentieren. Des Weiteren können solche Marker in einer Vielzahl in einem Hochdurchsatzverfahren parallel eingesetzt werden. Das Zuchtmaterial kann bereits im juvenilen Stadium auf die gewünschten bzw. interessanten Eigenschaften überprüft und für die weitere Bearbeitung sortiert werden. Ein Abwarten bis zur phänotypischen Merkmalsausprägung der Pflanze entfällt.

Kreuzungen, in denen Triticum timopheevii als Mutter und Triticum aestivum als Vater eingesetzt wurden, zeigten in manchen Nachkommen ein Ausbleiben der Pollenproduktion und der damit ausbleibenden Fertilität. Der Mechanismus der Fertilitätsrestauration basiert auf der Interaktion zwischen dem nukleären und mitochondrialen Genom. Grundsätzlich wird das Zytoplasma komplett mütterlich vererbt. Es findet keine Rekombination mit einer Kern-DNA statt. Das mitochondriale Genom enthält bei T.tomopheevii einen Sequenzbereich, der als orf256 bezeichnet wird. Dieser Bereich korreliert mit der Sterilität, wenn dieser durch Kreuzung im Zytoplasma von T. aestivum, das keine für Fertilitätsrestauration entscheidende Gene im Nukleus enthält, eingekreuzt wird (El-Shehawi et al., 2012). Es wird angenommen, dass ein dominantes Restorerallel (EP-A-16204599.1), das in einer R-Linie (Restorer) enthalten ist, mit einem Gen korreliert, das ein Protein kodiert, welches die mitochondriale Genexpression eines toxischen Proteins für die Fertilität reguliert (Kazama et al. 2008). Ist das Restorerallel rezessiv und der Phänotyp steril, trägt das mitochondriale Genom mit hoher Wahrscheinlichkeit das Sterilität induzierende orf256 aus T. timopheevii. Solche Linien werden in der Nomenklatur als A-Linien (CMS) bezeichnet, welche nur durch eine Rückkreuzung mit einer B-Linie (Maintainer) erhalten werden können (Becker 1993). Daraus erschließt sich, dass für die Produktion von CMS Saatgut immer ein Maintainer benötigt wird, wobei die Erstellung von Restorer-Saatgut diesen Zwischenschritt nicht benötigt.

Die aktuell am meisten erstellten Weizen-Hybriden basieren auf der Sterilisation der Mutterkomponente mit Gametoziden. Dies bringt den Vorteil, dass fast jede Zuchtlinie sofort und ohne weitere züchterische Bearbeitung chemisch sterilisiert werden könnte und die Kombinationen ohne aufwändige Arbeiten in Bezug auf steriles Plasma (A-Linie), Maintainer (B-Linie) oder Restorer (R-Linie) notwendig wären. Allerdings erfordert der Einsatz von Gametoziden unter anderem einen genauen Zeitpunkt der Applikation mit sehr engem Zeitfenster in Begleitung dafür unbedingt notwendiger günstiger Witterungsbedingungen, die bei Hybridherstellung mit A- und R-Linien wegfallen. Der Vorteil beim CMS-System bzw. bei der Arbeit mit R- und A-Linien ist, dass die A-Linie von Anfang an steril ist und man keine Selbstung erwartet, was beim Gemetozideinsatz nicht grundsätzlich auszuschließen ist. Umwelteinflüsse, technische und menschliche Fehler führen oft zu Fehlern in der Hybridproduktion bei Gametozidnutzung. Ein weiterer v.a. kostentechnischer Vorteil wäre der, dass man kein Gametozid für eine kommerzielle Hybridproduktion zulassen bräuchte.

Die Erstellung von Hybriden ohne Gametozideinsatz setzt voraus, dass das Material aus drei separaten Komponenten, nämlich den R-, B- und A-Linien besteht. Um diese drei Komponenten einwandfrei innerhalb des Zuchtprozesses unterscheiden bzw. identifizieren zu können, sind molekulare Marker und deren Kombination eine vielversprechende Methode und haben das Potential den Selektionsprozess enorm zu beschleunigen. Die Alternative wären aufwändige Kreuzungen und phänotypische Evaluationen des Materials, die neben Feldkapazitäten vor allem viel Zeit benötigen.

Eine Marker-gestützte Selektion zum Nachweis des sterilen Zytoplasmas in Kombination für Fertilitätswiederherstellung wäre wünschenswert, um ein zytoplasmatisches Sterilitätssystem zu vervollständigen und zu optimieren.

Erst mit Hilfe eines Markers für das Fremdplasma ist der praktische Einsatz eines Sterilitätssystems in einem Zuchtbetrieb sinnvoll und abgesichert.

Dies könnte helfen:
- Die praktischen Züchtungsprozesse bei der Hybridweizenzüchtung abzusichern
- Die Produktion von Hybridweizen abzusichern
- Zeit für aufwändige Tests durch notwendige Testkombinationen zu sparen.

### Aufgaben der vorliegenden Offenbarung

Es war somit eine der vorliegenden Anmeldung zugrunde liegende Aufgabe Mittel oder/und Screening-Verfahren bereitzustellen, die eine Grundlage für die Herstellung von Hybrid-Weizenpflanzen mit guten Ausbeuten ermöglichen.

Eine weitere Aufgabe war die Bereitstellung von Markern, die in Screening-Verfahren bei Weizenpflanzen verwendet werden können.

Eine weitere Aufgabe war es ein Verfahren bereit zu stellen, mit dem es möglich ist die Fertilität und Sterilität nachzuweisen und damit ein Mittel bereitzustellen, mit dem es möglich wird einfach, kostengünstig und/oder effizient A, Bund/oder R-Linien nachzuweisen und bereit zu stellen..

Eine weitere Aufgabe war es Marker oder/und ein Verfahren bereit zu stellen, mit dem es möglich ist, die zytoplasmatische Fertilität in Zellen, Pflanzen oder Pflanzenteilen zu bestimmen.

Eine weitere der vorliegenden Anmeldung zugrunde liegende Aufgabe war es, Mittel oder/und Screening-Verfahren bereitzustellen, die es ermöglichen das sterile vom fertilen Zytoplasma in Zellen, Pflanzen oder Pfalzenteilen, insbesondere in Weizenpflanzen, zu unterscheiden.

Insbesondere war es eine Aufgabe ein Mittel bereit zu stellen, womit es möglich wird die Unterscheidung vom "sterilen" Zytoplasma von T. timopheevii vom "fertilen" bzw. "normalen" Zytoplasma von T. aestivum zu bestimmen.

Eine weitere Aufgabe war die Bereitstellung von Markerkombinationen, die in Screening-Verfahren bei Weizenpflanzen verwendet werden können, um diese in eine entsprechende Hybrid-Elter-Komponente einteilen zu können, und insbesondere eine einfache Einteilung als R-, A- oder B-Linie zu ermöglichen.

### Beschreibung der Offenbarung

### Figurenbeschreibung

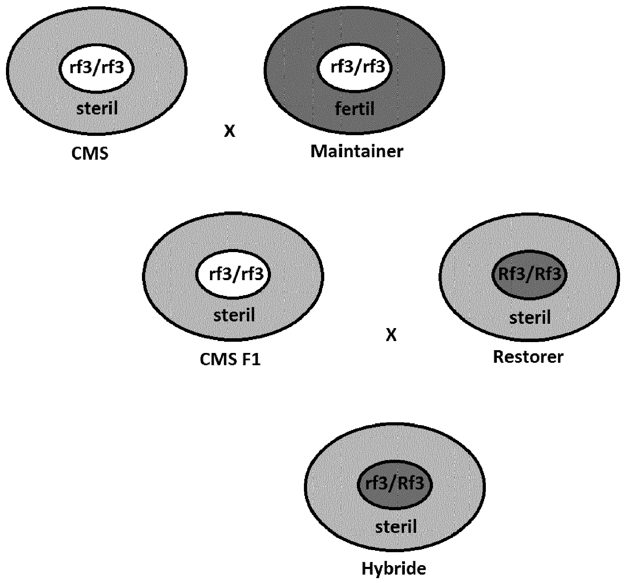

In Fig.1 wird die Kreuzungsfolge und der Zusammenhang der verschiedenen offenbarten Marker illustriert: rf/rf bzw. Rf/Rf bzw. rf/Rf ist im Kerngenom enthalten (QTL Marker); das Zytoplasma kann entweder steril (T. timophevii in hellgrau) oder fertil (T. aestivum in dunkelgrau) sein (CMS Marker).

### Technische Begriffe und Definitionen

Im Folgenden sollen einige Begriffe näher definiert werden. Im Übrigen sind die verwendeten Begriffe im Sinne der Erfindung nach dem allgemeinen Verständnis des Fachmannes zu verstehen.

"QTL T, QTL B und QTL W" im Sinne der Erfindung sind die Markergene Tdurum_contig50667_306 (QTL T), BS00011202_51 (QTL B) bzw. wsnp_CAP12_c4924_2241879 (QTL W) wie auch in Tabelle 1 dargestellt.

"CMS" ist die zytoplasmatische männliche Sterilität, die durch nicht zusammenpassen des Zytoplasma und der Kerngene zustande kommt. Meist verursacht durch extrem verschiedenes Material, das gekreuzt wurde. // "CMS" ist die zytoplasmatische männliche Sterilität, die durch nicht Kompatibilität des Zytoplasmas und der Kerngene zustande kommt. Meist verursacht durch genetisch extrem verschiedenes bzw. verwandtschaftlich sehr unterschiedliches Material, das gekreuzt wurde.

"R-Linie" oder "Restorer-Linie" besitzt mindestens ein Fertilitätswiederherstellungsgen, das bei einer Kreuzung mit einem männlichsterilen Genotyp die männliche Fertilität wiederherstellen kann. In diesem Zusammenhang wird auch von "Restorergen" gesprochen.

"cM" steht für centi Morgan. 1cM bedeutet so viel wie 1 Trennung von zwei Orten auf der DNS nach 100 Meiosen.

"BLAST" steht für "basic local alignment search tool" und wird für den Abgleich von Sequenzen zu einer Datenbank oder zu einer Referenzsequenz.

"Allel" bezeichnet eine Variante eines Locus auf einem Chromosom.

"Chromosom" bezeichnet eine cis-gekoppelte Ansammlung von Genen und Markern.

"QTL" steht für "quantitative trait locus". Das ist eine Chromosomen-Region, die eine signifikante Wahrscheinlichkeit aufweist, zur Ausprägung eines quantitativen phänotypischen Merkmals beizutragen.

"Transkript" ist eine in RNS umgeschriebene Sequenz eines Gens, die in eine Aminosäurensequenz übersetzt wird, welche wiederum ein Protein kodiert.

"Heterozygot" ist eine Allelkonstellation, die Mutterallele und Vaterallele gemischt enthält.

"Selbstung" beschreibt eine Methode, bei der die ausgewählte Pflanze nur den eigenen Pollen zur Bestäubung angeboten bekommt.

"F1" ist die erste filiale Generation, bei der die Allele der Mutterpflanze als auch der Vaterpflanze jeweils zu 50% das Genom nach deren Kreuzung (Gametenverschmelzung) kodieren.

"F2" ist eine zweite filiale Generation, der die erste vorangeht (F1). Bei den Pflanzen wird die F1 geselbstet, was zu einer Aufspaltung führt. Nach der zweiten Mendelschen Regel spaltet sich sowohl der Genotyp als auch der Phänotyp auf.

"Rf" bedeutet "restoration of fertility" und wird in dieser Beschreibung als ein Locus für die Fertilitätswiederherstellung auf einem Chromosom verwendet.

"Chi-quadrat-Test" steht für eine statistische Methode mit der eine beobachtete Verteilung eines Merkmals mit einer vorausgesagten verglichen wird. Dabei wird ein Wert generiert, der zur Aussage über die Signifikanz der beobachteten Verteilung herangezogen wird.

"SNPChip" steht für ein Verfahren, bei dem kurze Sequenzen auf einem Trägerobjekt aufgetragen werden um Polymophismen, auf der DNS verteilt, zu detektieren.

"CI" steht für confidence intervall und definiert das Vertrauensintervall der flankierenden Marker eines QTL.

"Genetische Karte" ist eine Zusammenfassung der errechneten Rekombinationshäufigkeiten zwischen den Markern, die in centi Morgan (cM) angegeben werden.

"Umwelteinflüsse", in diesem Kontext sind hauptsächlich die Witterungsbedingungen gemeint, welche die Sterilität verursachen können.

"Fertilität" bedeutet Pflanzen und insbesondere bei Weizen die Tatsache, dass der in den Antheren vorhandene Pollen lebensfähig und befruchtungsfähig ist.

"Sterilität" dagegen bedeutet bei Weizen die Tatsache, dass zwar in der Blüte die Antheren vorhanden sind, dass diese aber keinen lebensfähigen bzw. befruchtungsfähigen Pollen enthalten.

"Fertilitätsbestimmung" im Sinne der Erfindung ist der Nachweis inwiefern eine Zelle, Pflanze oder Pflanzenteil fertil sind.

"Fertilitätswiederherstellung" ist der Vorgang, bei dem das/die Restorer-Gen/e dazu beiträgt/beitragen, dass die Nachkommen einer sterilen Pflanze fertil sind.

"Allellink (LD /linkage disequilibrium)" gekoppelte Abschnitte auf dem Chromosom, die zu einem nachweisbaren Anteil an die Nachkommen weitervererbt werden.

"B-Linie" oder "Maintainer-Linie" erhält die Pollensterilität im Kerngenom. Enthält ein normales Zytoplasma, das Pollenfertilität aufrechterhält.

"A-Linie" oder "CMS-Linie" ist eine vollständig sterile Pflanze, zurückzuführen auf das Vorhandensein eines sterilen Zytoplasmas ohne jedes die Fertilität wiederherstellendes Allel im Kerngenom.

"Molekulare Marker" oder "Marker" werden in der Genetik eingesetzt um bestimmte Allele im Genom zu "markieren". Dies kann dazu genutzt werden, um eine Aussage über einen Allel gekoppelten Phänotyp treffen zu können.

"Zytoplasma" ist der Überbegriff für das zelluläre Innere, das feste und flüssige Kompartimente enthält. Enthält u.a. auch Organellen mit RNA, aber keinerlei Kern-DNA.

"Kerngenom" ist der Überbegriff für die im Zellkern vorhandenen Chromosomen bzw. Kern- DNA.

"Hybride" wird in der Züchtung das Material bezeichnet, das aus der Kreuzung einer mütterlichen und einer väterlichen Komponente entsteht.

"Hybridsorten" werden Sorten in der Züchtung bezeichnet, bei denen mit Hilfe eines Sterilitätssystems (mechanisch, chemisch oder biologisch = cms) Hybridsaatgut in großer Menge produziert werden können. Dieses Saatgut ist gleichzeitig das gewünschte Endprodukt, das dem Landwirt verkauft wird.

"Genotyp" repräsentiert eine individuelle Allel- und Genkonstellation.

"Phänotyp" beschreibt die Ausprägung der Merkmale einer Pflanze.

"Gametozid" ist eine Chemikalie, die im Weizen eingesetzt werden kann, um die Entwicklung der männlichen Blütenteile zu verhindern und somit diese zu sterilisieren.

"Polymorph" bedeutet, dass an einer Stelle im Genom zwei Varianten von Allelen vorkommen können. Z.B.: das A Allel kommt von der Mutter und das B Allel kommt von dem Vater. Die F1 Nachkommen aus dieser Kreuzung tragen in einer Pflanze auf einem der Chromosomen das Allel A und auf dem anderen das B Allel.

Nach IUPAC Nukleotid Kodierung steht das "R" für den Polymorphismus A/G.

Nach IUPAC Nukleotid Kodierung steht das "Y" für den Polymorphismus C/T.

Nach IUPAC Nukleotid Kodierung steht das "S" für den Polymorphismus G/C.

KASP steht für Kompetitive Allelspezifische PCR und wird für die Fluoreszenz basierte Genotypisierung eingesetzt. Die PCR Protokolle wurden von der LGCGroup (www.lgcgroup.com) bereitgestellt.

Im Folgenden wird die Erfindung und ihre verschiedenen Ausgestaltungen weiter beschrieben.

In einem Aspekt betrifft die Erfindung einen CMS Marker, der durch bestimmte Merkmale und insbesondere Sequenzmerkmale charakterisiert ist. Mögliche Sequenzmerkmale sind aus der vorliegenden Offenbarung ersichtlich und insbesondere sind diese aus der hierin enthaltenen Sequenzübersicht und dem Sequenzprotokoll ersichtlich, auf die hiermit verwiesen wird.

In einem Aspekt betrifft die Erfindung ein CMS Markergen charakterisiert durch die Sequenz SEQ ID NO.5, ein Fragment davon mit mindestens 12 Basenpaaren (bp) oder mit 12 bis 30 bp, oder eine Primersequenz aufweisend mindestens 12 Basenpaaren (bp) oder aufweisend 12 bis 30 bp , der Sequenz SEQ ID NO.5, vorzugsweise SEQ ID NO.9, 10, und 11, die aufweist drei Polymorphismen gekennzeichnet durch zwei R- (A/G) und einen Y-Polymorphismus (C/T) und gegebenenfalls einen S-Polymorphismus (G/C) und/oder ein Y-Polymorphismus (C/T) (z. B. SEQ ID NO.6).

In einem Aspekt betrifft die Erfindung ein CMS Markergen charakterisiert durch die Sequenz SEQ ID NO.5, ein Fragment davon mit mindestens 12 Basenpaaren (bp) oder mit 12 bis 30 bp, oder eine Primersequenz aufweisend mindestens 12 Basenpaaren (bp) oder aufweisend 12 bis 30 bp, der Sequenz SEQ ID NO.5, vorzugsweise SEQ ID NO.9, 10, und 11, die aufweist drei Polymorphismen gekennzeichnet durch zwei R- (A/G) und einen Y-Polymorphismus (C/T) und gegebenenfalls einen S-Polymorphismus (G/C) und/oder ein Y-Polymorphismus (C/T) (z.B. SEQ ID NO.6) zur Verwendung in einem Verfahren zur Bestimmung des Zytoplasmas einer Weizenpflanze, Teilen einer Weizenpflanze oder/und einer Weizenpflanzenzelle, vorzugsweise wobei die Weizenpflanze, Teilen einer Weizenpflanze oder/und die Weizenpflanzenzelle ein T. timopheevii oder ein T. aestivum ist.

In einem Aspekt betrifft die Erfindung eine Primersequenz geeignet zum Nachweis einer Sequenz in einer Weizenzelle (Triticae spec.), gekennzeichnet durch mindestens 12 Basenpaaren (bp) oder durch 12 bis 30 bp aus der Sequenz SEQ ID NO.5, vorzugsweise SEQ ID NO.9, 10, und 11, und die aufweist zwei R-Polymorphismen (A/G) und einen Y-Polymorphismus (C/T) und gegebenenfalls einen S-Polymorphismus (G/C) und/oder ein Y-Polymorphismus (C/T) (z. B. SEQ ID NO.6).

In einem Aspekt betrifft die Erfindung ein Verfahren zur Bestimmung des Zytoplasmas einer Weizenpflanze, Teilen einer Weizenpflanze oder/und einer Weizenpflanzenzelle, vorzugsweise wobei die Weizenpflanze, Teilen einer Weizenpflanze oder/und die Weizenpflanzenzelle ein T. timopheevii oder ein T. aestivum ist, wobei mindestens eine Primersequenz gemäß Anspruch 3 verwendet wird.

In einem Aspekt betrifft die Erfindung einen Kit umfassend mindestens eine Primersequenz wie hierin beschrieben und gegebenenfalls eine Polymerase, Nucleotide und Puffer.

In einem Aspekt betrifft die Erfindung einen Schnelltest zum Nachweis von Zytoplasma in einer Probe, umfassend die Schritte Verwendung von Komponenten umfassend mindestens CMS Markergen charakterisiert durch die Sequenz SEQ ID NO.5, ein Fragment davon mit mindestens 12 Basenpaaren (bp) oder mit 12 bis 30 bp, oder eine Primersequenz aufweisend mindestens 12 Basenpaaren (bp) oder aufweisend 12 bis 30 bp , der Sequenz SEQ ID NO.5, vorzugsweise SEQ ID NO 9, 10, oder 11, Temperieren, vorzugsweise Aufheizen der Komponenten bis zu einer Aufschmelztemperatur, Temperieren, vorzugsweise Abkühlen bis auf eine Hybridisierungstemperatur, und Durchführen einer Visualisierungsreaktion zur Sichtbarmachung des Vorhandensein von Zytoplasma von T. timopheevii oder von T. aestivum. In einem Aspekt betrifft die Erfindung ein oder mehrere Markergene oder eine Kombination davon, vorzugsweise mindestens 2 oder 3 Markergene, wobei das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm in Kombination mit einem CMS Markergen wie oben beschrieben oder einer Primersequenz wie oben beschrieben zur Verwendung in einem Verfahren zur Bestimmung der Fertilität oder/und der Fertilitätswiederherstellung oder/und von Restorergenen oder/und von ausgewählten Allelen oder einer Allelkombination in einer Weizenpflanze oder Teilen davon oder in einer Weizenzelle.

Das Markergen oder die Markergene oder die Kombination von Markergenen wie oben beschrieben kann optional ausgewählt sein aus der Gruppe bestehend aus den Markern QTL T, QTL B und QTL W.

In einem weiteren Aspekt betrifft die Erfindung ein Verfahren zum Nachweis der Fertilität oder/und der Fertilitätswiederherstellung oder/und von Restorergenen in einer Weizenpflanze oder Teilen einer Weizenpflanze oder einer Weizenzelle, wobei ein genetischer Marker oder eine Kombination von genetischen Markern verwendet wird ausgewählt aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm, das Markergen oder die Markergene vorzugsweise ausgewählt werden aus der Gruppe bestehend aus QTL T, QTL B und QTL W, und die Fertilität oder der Grad der Fertilität bestimmt wird in Kombination mit einem CMS Markergen wie oben beschrieben oder einer Primersequenz wie oben beschrieben.

In einem weiteren Aspekt betrifft die Erfindung ein Verfahren zum Nachweis eines ausgewählten Allels oder/und einer ausgewählten Allelkombination in einer Weizenpflanze oder Teilen einer Weizenpflanze, wobei ein genetischer Marker oder eine Kombination von genetischen Markern ausgewählt aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm oder ausgewählt aus der Gruppe QTL T, QTL B und QTL W, verwendet werden, in Kombination mit einem CMS Markergen wie oben beschrieben oder einer Primersequenz wie oben beschrieben und die ausgewählten Allele bestimmt werden.

### Cl 11: In noch einem weiteren Aspekt betrifft die Erfindung ein

Verfahren zur Selektion einer Pflanze, die ein oder mehrere funktionelle Restorergene für eine zytoplasmatische männliche Sterilität (CMS) in Weizen aufweist, umfassend die Schritte:
a.Screenen einer Pflanzenpopulation oder einer Sammlung oder Gruppe von Pflanzen für mindestens einen oder zwei Marker in Kombination mit einem CMS Markergen wie oben beschrieben oder einer Primersequenz wie oben beschrieben, vorzugsweise drei Marker, wobei die Markernukleinsäure ein Allel umfasst, das einen Allellink (LD /linkage disequilibrium) mit einem oder mehreren Restorergenen darstellt oder sich in einem oder mehreren Restorergenen befindet,
b.Nachweisen der Markernukleinsäuren,
c.Identifizieren der Pflanzen, die die Markernukleinsäuren aufweisen,
d.Selektion der Pflanzen, die die Markernukleinsäuren aufweisen.

Dieses Verfahren kann die zusätzlichen Schritte umfassen: a. Erhalten der Pflanze, die ein oder mehrere Restorergene enthält, b. Kreuzen der Pflanze mit einer zweiten Pflanze, die ein oder mehrere Restorergene enthält, um Nachkommenpflanzen zu erhalten, c. Screenen der Nachkommenpflanzen für zwei oder drei der Marker aus der Gruppe, bei der das Markergen mit einem oder mehreren Restorergenen assoziiert ist (Vertrauensintervall) innerhalb von 17 Mb auf Chromosom 1B oberer Arm, innerhalb von 5 Mb auf Chromosom 6A unterer Arm oder/und innerhalb von 1 Mb auf Chromosom 6A oberer Arm oder für zwei oder drei der Marker QTL T, QTL B und QTL W, d. Selektion der Pflanzen, die eine oder mehrere Markernukleinsäuren ausgewählt von QTL T, QTL B und QTL W aufweist.

In diesem Verfahren nach können die Schritte a.) bis d.) wiederholt werden, um eine Pflanze zu erhalten, die alle drei der Marker aus der Gruppe, bei der das Markergen mit einem oder mehreren Restorergenen assoziiert ist (Vertrauensintervall) innerhalb von 17 Mb auf Chromosom 1B oberer Arm, innerhalb von 5 Mb auf Chromosom 6A unterer Arm oder/und innerhalb von 1 Mb auf Chromosom 6A oberer Arm aufweist oder die alle drei der Marker QTL T, QTL B und QTL W aufweist (Pyramidisierung).

In noch einem weiteren Aspekt betrifft die Erfindung die Verwendung von einem oder mehreren genetischen Markern wie oben beschrieben zum Screenen oder zur Selektion von Zellen, Pflanzen oder/und zur Pyramidisierung.

In der erfindungsgemäßen Marker-Kombination oder dem erfindungsgemäßen Verfahren kann die Kombination die Marker QTL T und QTL B, oder QTL T und QTL W umfassen oder aus ihr bestehen bzw. das erfindungsgemäße Verfahren eine Kombination der Marker QTL T und QTL B, oder der Marker QTL T und QTL W oder der Marker QTL T, QTL B und QTL W verwenden.

Bei der erfindungsgemäßen Marker-Kombination oder dem erfindungsgemäßen Verfahren kann einer der Marker auf Chromosom 1B liegen.

Überraschender Weise konnten bereits mit einem der erfindungsgemäßen Marker oder einer Kombination von zwei Markern Pflanzen identifiziert werden, die eine verbesserte Fertilität aufweisen. Vorteilhafter Weise konnten so für Hybrid-Sorten geeignete Pflanzen identifiziert werden.

Es war überraschend, dass der erfindungsgemäße Marker, der mit Chromosom 1B assoziiert ist dazu geeignet ist die Fertilität in einer Weizenpflanze zu einem hohen Prozentsatz korrekt zu bestimmen (Tabelle 2).

In 180 heterozygoten Testpflanzen für den Marker QTL T auf Chromosom 1B konnten beispielsweise 75% der fertilen Pflanzen mit dem Marker assoziiert werden. QTL T konnte im homozygoten Zustand mit dem Allele T 96% der vollfertilen detektieren (Tabelle 2). Zu einer Kombination von heterozygoten Marker QTL T und homozygoten Alleles für Fertilitätswiederherstellung des QTL W konnten bereits 95% der vollfertilen Genotypen assoziiert werden. Wenn der Marker QTL W durch den Marker QTL B ausgetauscht wurde, konnte 98% der Marker Allele Kombination mit dem Phänotyp assoziiert werden (Tabelle 4). Eine 100%-ge Fertilitätswiederherstellung konnte mit der Kombination der drei Marker beobachtet werden (Tabelle 5).

Die Erfindung stellt mit einer Allelkombination des fertilen Elters auf zwei unterschiedlichen Chromosomen ein neuartiges System dar, das in so einer Weise noch nirgendwo beschrieben wurde.

Ein weiterer Vorteil des erfindungsgemäßen Verfahrens und der erfindungsgemäßen Kombination von Markern ist, dass die Anreicherung der Fertilitätswiederherstellenden Allele mit diesen drei Markern überprüft werden kann.

Weitere Vorteile des erfindungsgemäßen Verfahrens und der erfindungsgemäßen Kombination von Markern ist auch, dass eine Detektion von sterilen CMS Linien mittels der drei molekularen Marker auch möglich ist, wenn es im spaltenden Restorermaterial auf die entsprechenden Allele selektiert wird (Tabelle 5). Weiterhin können auch Maintainer selektiert werden, wenn der Phänotyp eines Genotyps fertil ist, trotz der drei Allele für Sterilität (CCC) wie in Tabelle 5 aufgeführt. Das ist der Fall, wenn das Zytoplasma die Fertilität verursacht. Weiterhin von Vorteil ist, dass die Fertilitätswiederherstellung in homozygoter Form für die drei Marker nicht von Umweltfaktoren beeinflusst wird. Schließlich wird es mit dem erfindungsgemäßen Verfahren und Markerkombination möglich eine wirtschaftlich interessante Hybridproduktion mit diesem Systemansatz anzustreben.

Der erfindungsgemäße Markereinsatz für die Fertilitätswiederherstellung erlaubt eine präzise Produktion von Restorer-Linien, die eine wirtschaftliche Produktion von Hybridsaatgut mit einer entsprechenden CMS-Linie rentabel machen kann. Im Übrigen sind die oben beschriebenen Merkmale in der technisch möglichen Art und Weise kombinierbar auch wenn dies in den Ausführungen oben und in den Beispielen nicht explizit beschrieben wurde.

### Kombination von QTL und CMS Marker gemäß der Offenbarung

In einem Aspekt betrifft die Erfindung ein Markergen zur Verwendung in einem Verfahren zur Bestimmung von sterilem oder fertilem Zytoplasma in einer Zelle, Weizenpflanzenzelle, Weizenpflanze oder Teilen davon. Das CMS Markergen gemäß der Erfindung ist der Marker TaMT CMS.

In einem weiteren Aspekt betrifft die Erfindung ein Verfahren zum Nachweis des sterilen oder fertilen Zytoplasmas in einer Weizenpflanze oder Teilen einer Weizenpflanze, wobei ein genetischer Marker verwendet wird.

In einem weiteren Aspekt betrifft die Erfindung ein Verfahren zum Nachweis eines ausgewählten Allels in einer Weizenpflanze oder Teilen einer Weizenpflanze, wobei ein genetischer Marker verwendet wird und das ausgewählte Allel bestimmt wird.

In noch einem weiteren Aspekt betrifft die Erfindung ein Verfahren zur Selektion einer Pflanze, die ein fertiles oder steriles Zytoplasma aufweist, die folgenden Schritte umfassend:
a. Screenen einer Pflanzenpopulation oder einer Sammlung oder Gruppe von Pflanzen für den TaMT_CMS Marker, wobei die Markernukleinsäure ein Allel umfasst, das für fertiles oder steriles Zytoplasma kodiert,
b. Nachweis der Markernukleinsäuren,
c. Identifizieren der Pflanzen, welche die Markernukleinsäuren aufweisen,
d. Selektion der Pflanzen, welche die Markernukleinsäuren aufweisen.
e. Einteilung der Pflanzen in T. timophevii oder T. aestivum Zytoplasma.

Des Weiteren kann der TaMT_CMS Marker in Kombination mit den Markern für Fertilitätswiederherstellung in folgenden Schritten eingesetzt werden und umfasst:
a. Screenen einer Pflanzenpopulation oder einer Sammlung oder Gruppe von Pflanzen für den TaMT_CMS Marker in Kombination mit Tdurum_contig50667_306 (Rf3) Marker, wobei die Kombination aus dem Allel für Fertilität bzw. Sterilität durch Rf3 und dem Allel für fertiles oder steriles Zytoplasma durch TaMT_CMS kodiert,
b. Einteilung der Hybrid-Elter-Komponente in A-, B- und R-Linien

Die Kreuzungsfolge und der Zusammenhang der verschiedenen hier offenbarten Marker wird in Fig. 1 illustriert und ist daruas ersichtlich. rf/rf bzw. Rf/Rf bzw. rf/Rf ist im Kerngenom enthalten (QTL Marker). Das Zytoplasma kann entweder steril (T. timophevii in hellgrau) oder fertil (T. aestivum in dunkelgrau) sein (CMS Marker).

Unter Verwendung der erfindungsgemäßen Marker oder/und dem erfindungsgemäßen Verfahren kann das sterile Zytoplasma so in einfacher Weise von fertilem Zytoplasma unterschieden werden.

Das sterile und fertile Zytoplasma kann aufgrund der vorliegenden Offenbarung und mit den erfindungsgemäßen Markern nunmehr im Wesentlichen exakt bestimmt werden.

Ein weiterer Vorteil des erfindungsgemäßen Verfahrens und der Kombination von TaMT_CMS mit dem Marker Tdurum_contig50667_306 (Rf3) erlaubt die vollständige Einteilung der A-, B- und R-Linien in deren entsprechende Hybrid-Elter-Komponente.

In einem Validierungstest konnten durch die Markerkombination aus Tdurum_contig50667_306 (Rf3) und TaMT_CMS folgende Einteilungen vorgenommen werden:
Aus 380 Genotypen wurden 55 als bekannte T. aestivum Maintainer Linien als diese auch detektiert. Zusätzlich konnten 25 weitere Genotypen mit dem T. aestivum Zytoplasma ausgemacht werden können, die allerdings als Restorer geführt werden. Diese nutzen das Rf3 nicht für die Fertilitätswiederherstellung. Somit würden diese 25 das Restorerzuchtprogramm in dieser Form verlassen. Des Weiteren wurden 11 vollständig sterile Genotypen mit rezessivem Rf3 Allel und T. timopheevii Zytoplasma ausgemacht werden können. Die übrigen 289 Genotypen waren wie erwartet mit dem dominanten Rf3 Allel und dem T. timopheevii Zytoplasma detektiert worden.

Unter praktisch züchterischen Gesichtspunkten werden mit den nunmehr hier offenbarten Markern Werkzeuge für eine erfolgreiche Hybridzüchtung bei Weizen auf Basis des sterilen Plasmas von Triticum timopheevii bereitgestellt und komplettiert. Die Kombination der verschiedenen offenbarten Marker zusammen ermöglichen eine überlegene Bestimmung des genetischen Sachverhalts in den Zellen bzw. Pflanzen oder Pflanzenteilen und somit ein zuverlässiges Mittel für die Selektion sowie Hybridsamenherstellung.

Zusammenbau von SEQ ID NO.5 erfolgte aus der Kombination von SEQ ID NO. 4 und den Sequenzen SEQ ID NO.7 und SEQ ID NO.8. SEQ ID NO.7 enthält eine sehr ähnliche Sequenz zu SEQ ID NO.4 (88%). Des Weiteren ist ein Teilabschnitt der SEQ ID NO.4 mit einer 99% Übereinstimmung zu SEQ ID NO.8 vorhanden. Ein Abschnitt SEQ ID NO.5 konnte ausfindig gemacht werden, der zwei Polymorphismen zwischen SEQ ID NO.8 und SEQ ID NO.5 enthält. Diese beiden Polymorphismen konnten zur speziellen KASP Assay Erstellung eingesetzt werden, um die SEQ ID NO.8 auf Chr3B liegend von der mitochondrialen Sequenz SEQ ID NO.5 unterscheiden zu können.

Des Weiteren enthält SEQ ID NO.7 diese beiden Polymorphismen nicht, aber einen Polymorphismus, der sowohl für die Unterscheidbarkeit zwischen SEQ ID NO.7 und SEQ ID NO.5 verantwortlich ist, als auch für die KASP Assay Erstellung SEQ ID NO.9, SEQ ID NO.10 und SEQ ID NO.11 noch in einem möglichen Bereich liegt.

Mögliches Schnelltestverfahren für den Nachweis von T. timopheevii bzw. T. aestivum können folgende Schritte beinhalten:
- Blattmaterial wird einer Weizenpflanze entnommen
- Das Blatt wird zerquetscht oder püriert oder mit einer anderen Methode werden die Zellen aufgebrochen
- Durch Zugabe einer Chemikalie oder mittels eine Filtration könnte der Zellsaft mit der darin enthaltenen DNS gewonnen werden
- Die SEQ ID NO.5 oder die Primer SEQ ID NO.9 und SEQ ID NO.11 könnten mit einer stabilisierenden Chemikalie mit der gewonnen Zellsaft vermengt werden
- Eine Vorrichtung, in der das Gemisch aufgeheizt werden kann, um die DNS Doppelstränge zu Einzelsträngen aufzuschmelzen wird benötigt
- Danach wird die Temperatur abgesenkt, damit eine Primer - DNS Hybridisierung erfolgen kann
- Nach einer erfolgreichen Primer - DNS Hybridisierung erfolgt die Auslesung der Fluoreszenz mit einer entsprechenden Vorrichtung sowie der entsprechenden Wellenlänge

Ein Nachweis T. timopheevii bzw. T. aestivum durch Sequenzierung kann z.B. so erfolgen:
- Das orf256 von T. timopheevii kann mittels Sequenzierung der mitochondrialen DNS nachgewiesen werden. Es befindet sich im T. timopheevii vorgeschaltet vor dem coxI. Im T. aestivum fehlt orf256.

Die Erfindung soll durch die folgenden Beispiele illustriert werden ohne dass diese als Einschränkung verstanden werden sollen.

### Beispiele

### A. QTL Marker und ihre Verwendung1. Pflanzen Material und Vorgehen zur Identifizierung der erfindungsgemäßen Marker

Der Elter, der als Mutter für die Kreuzung eingesetzt wurde, wurde kastriert (P1). Dieser Elter enthält das für die Sterilität induzierende Zytoplasma von Triticum Timopheevii. Des Weiteren enthält dieser Elter (P1) das/die entsprechende/n Rf Gen/e für die Fertilitätswiederherstellung.

Der P1 wurde mit einem Maintainer (P2 ohne Fertilitätswiederherstellende Gene) gekreuzt, um (I) die Rf Gene heterozygot in der F1 Hybride zu erhalten und (II) das steril induzierende Zytoplasma nicht zu wechseln. Denn das Zytoplasma wird immer von der Mutter an die Nachkommen weitervererbt.

Die Hybride (F1) wurde geselbstet (> 1 F1 Pflanzen eingesetzt), um eine phänotypische Aufspaltung in der F2 Generation für die Fertilitätswiederherstellung zu erhalten. Die F2 Population wurde im Feld dann angezogen.

### 2. Durchführung der Phänotypisierung

Die Bonitur erfolgte für vier F2 Populationen, die aus vier unterschiedlichen Elternpaarungen bestanden, wobei die vier Mütter auf den gleichen Ursprung für Fertilitätswiederherstellung zurückgehen.

Danach erfolgte eine Bonitur: vollfertil, teilfertil(∼50% fertil), spitzensteril (∼75% fertil), vollsteril.

Die phänotypischen Merkmale der Fertilitätswiederherstellung aller voll-, spitzen- und teilfertilen F2 Genotypen wurden zu einer Gruppe zusammengefasst ("fertil") und die vollsterilen Pflanzen zu der anderen Gruppe zugeordnet. Zwei von vier Populationen zeigten eine 3:1 Verteilung (fertil:steril), die andeutete, dass das/die Rf Gen/e eine dominante Wirkung auf die Fertilitätswiederherstellung hat/haben und dass ein Hauptgen mit einem großen phänotypischen Effekt an der Fertilitätswiederherstellung beteiligt ist. Von den beiden 3:1 verteilten Populationen wurde die anhand des chi-quadrat-Tests am besten verteilte für die Genotypisierung eingesetzt.

### 3. Genotypisierung und Erstellung der genetischen Karte

Die Genotypisierung von 346 F2 Genotypen und der entsprechenden Eltern erfolgte bei der Firma TraitGenetics (Gatersleben, Deutschland) mittels einem 15K SNPChip für Weizen.

Von den 13006 SNP-Markern konnten 3695 polymorphe zwischen den beiden Eltern der genotypisierten F2 Population extrahiert und für weitere Analysen eingesetzt werden. Die Zuordnung zu den Kopplungsgruppen/Chromosomen wurde für alle polymorphe Marker mittels BLAST und der aktuell verfügbaren physikalische Assemblierung (09.08.2016 Triticum aestivum.TGACv1.dna.toplevel.fa.gz) des Weizengenoms ermittelt (Gramene).

Marker, welche zu keinem Chromosom annotiert werden konnten, wurden manuell unter der Zuhilfenahme des Webtools https://triticeaetoolbox.org entsprechendem Chromosom zugewiesen. Marker ohne eine konkrete Chromosomzuweisung wurden von weiteren Analysen ausgeschlossen. Von den 3695 SNP-Markern waren 1694 für die genetische Kartenerstellung redundant. Mit 2001 SNP-Markern wurde eine genetische Karte für die 21 Weizen-Chromosome berechnet. Dafür wurde die freiverfügbare Software Carthagene (De Givry et al., 2004) benutzt.

### 4. QTL-Detektion

Die QTL Detektion erfolgte mit der Statistik-Software R unter dem Einsatz des R Packages R/qtl wie in Benke et al. (2014) beschrieben. Es wurden 3 signifikante QTLs detektiert: 1 auf Chromosom 1B und 2 auf Chromosom 6A (Tabelle 1).

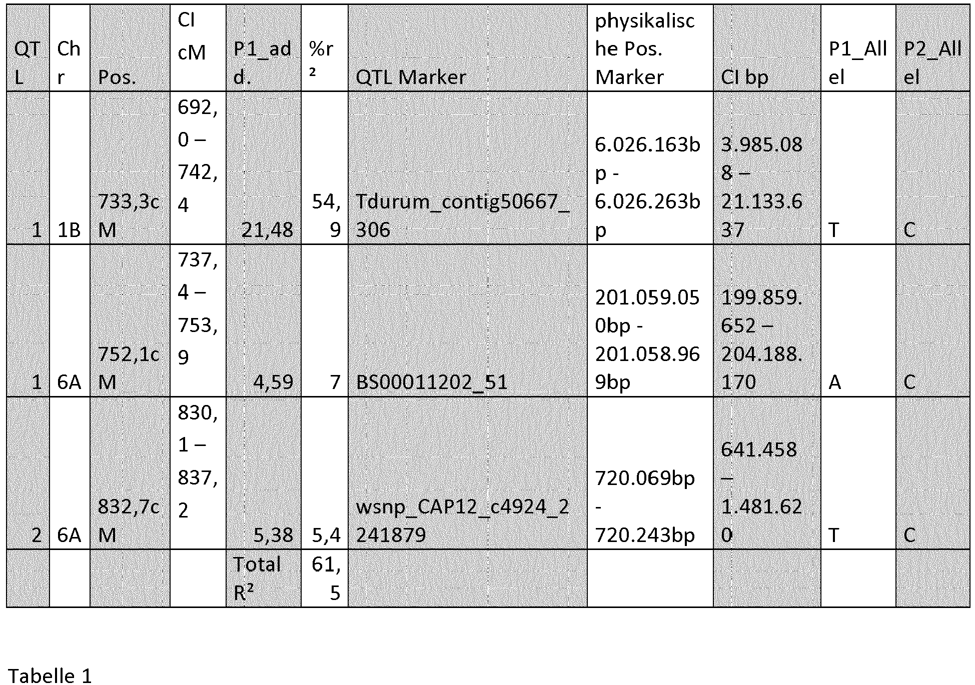

Von vorangegangenen publizierten Studien wurde bereits gezeigt, dass Chromosom 1B das Gen Rf3 beherbergt (Ahmed et al. 2001), welches als ein Hauptgen für die Fertilitätswiederherstellung dient. Allerdings wurden bisher weder die exakte physikalische Lokalisierung noch präzise Marker gezeigt. Genau dasselbe gilt für 6A, wo Rf6 lokalisiert wurde (Rongxia et al., 2002).

Erfindungsgemäß wurden 3 QTL und die entsprechenden Marker detektiert (Tabelle 1-5).

Der Einsatz von einem dieser Marker für die Fertilitätsvoraussage gibt bereits einen Hinweis beim Fertilitätsnachweis; eine verbesserte Aussage kann mit zwei der erfindungsgemäßen Marker gemacht werden. In Kombination zeigen zwei und besser alle drei erfindungsgemäßen Marker eine präzise Voraussage der Fertilität in der Genetik, wenn die Allele "T", "A", und "T" des P1 Elter zusammen auftreten. Es ist naheliegend, dass diese Allelkombination widerstandsfähiger gegenüber der Teil-, Spitzen- und Vollsterilität ist, die von Umwelteinflüssen verursacht wurde. Ein entsprechender Nachweis für die F3 Population kann auch durchgeführt werden.

Es konnte gezeigt werden, dass wenn der Marker auf Chromosom 1B homozygot für T oder heterozygot ist und die beiden Marker auf Chromosom 6A homozygot für T und A Allele sind, werden die Genotypen vollständig fertil sein (Tabelle 5).

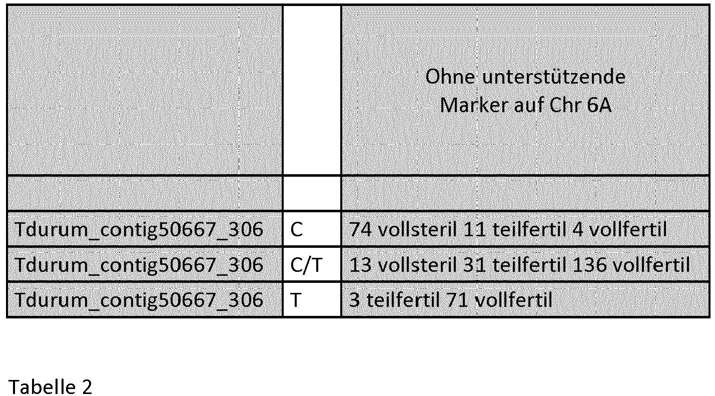

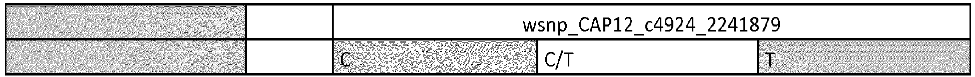

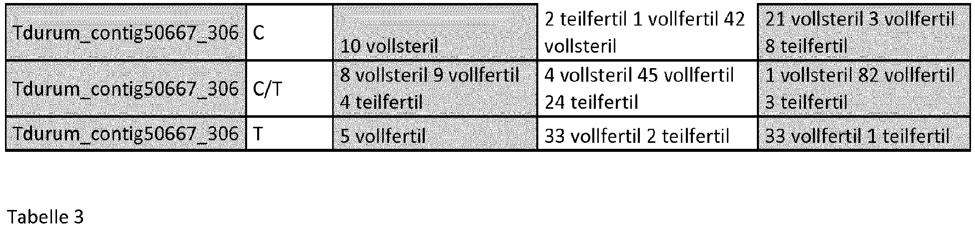

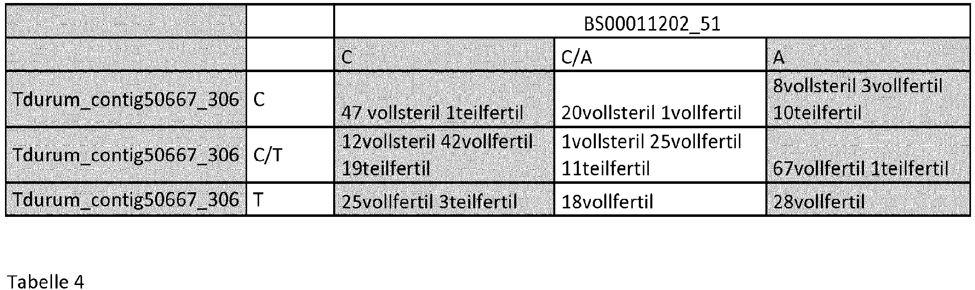

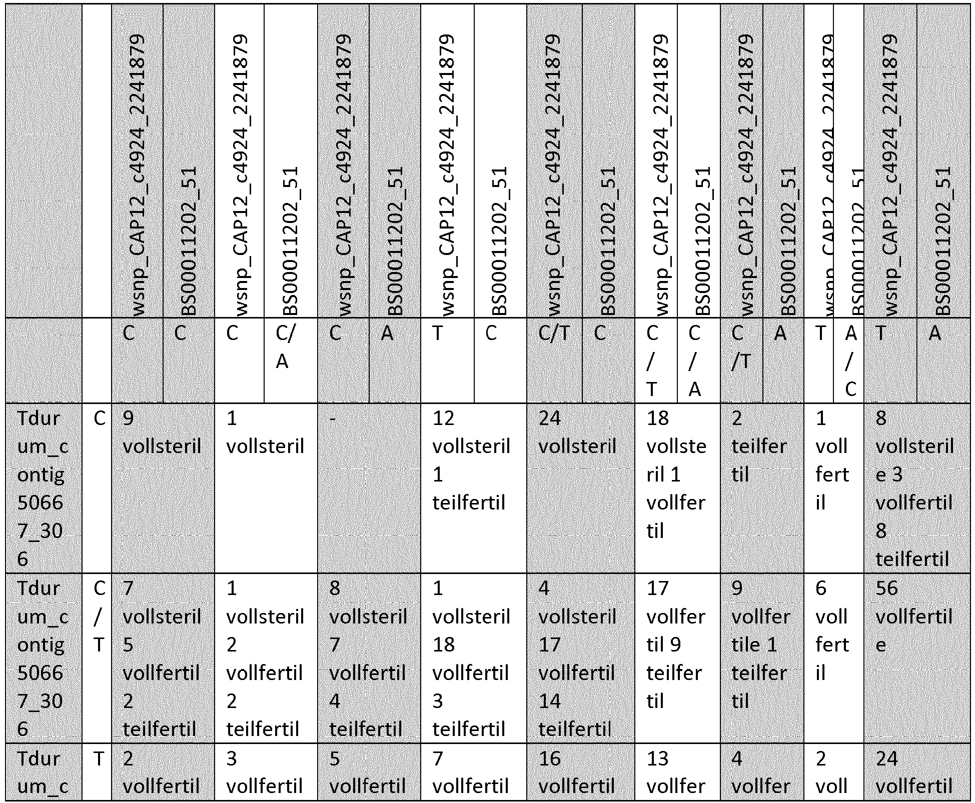

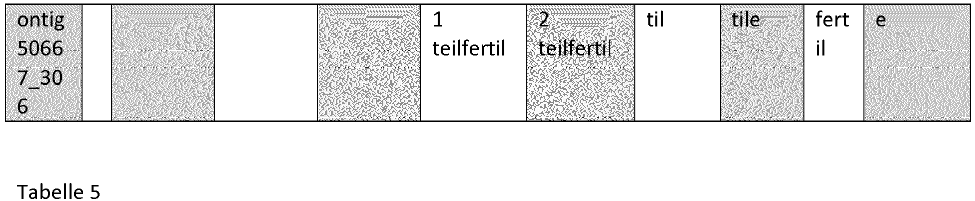

Im Folgenden sind die QTL Markersequenzen der vorliegenden Offenbarung aufgelistet.

**Tabelle 6**

| Name | Chromosom | Sequenz |
|---|---|---|
| Tdurum_ contig50 667_306 QTL T SEQ ID NO.1 | 1B | CGACGACGACGAGGATGCCGAGTTTGATGACATGGAGGATTATATCGACG[A/G]cgcggact gggacgccgacatgtatgatgatgtgttcgatgtctgaagga |
| wsnp_C AP12_c4 924_224 1879 QTL W SEQ ID NO.2 | 6A | TAGTTTTCTTCTTGGTTGCCTGTAATAACATCATAGGTTGAGGTTACCAGCCGCTCCGCATA AAGCTTGAAAGTTGAACTTGCTGAAGCGGCGAGCCTCG[C/T]GAGCACCACCACCACCGAC AAGTATCAGGAGCACCTTTCCCTCCAGTACATGTAAATAAAAAGGAGTATACATATTTGTCC TATGTATCGATTTGAGAAT |
| BS00011 202_51 QTL B SEQ ID NO.3 | 6A | AACTTTATGCATGCGCCATTGCAGATCAATATCTAGTAGATCAATCAGCT[G/T]ctctcgcctaa caggatcaactccatcaactccaaatatcatgtgctcag |

### B. QTL und CMS Marker sowie ihre Verwendung

Im Folgenden sind die QTL und CMS Markersequenzen der vorliegenden Offenbarung aufgelistet.

**Tabelle 7: QTL und CMS Markersequenzen der Erfindung**

| Name | Chromosom | Sequenz |
|---|---|---|
| Tdurum_ contig50 667_306 QTL T SEQ ID NO.1 | 1B | CGACGACGACGAGGATGCCGAGTTTGATGACATGGAGGATTATATCGACG[A/G]cgc ggactgggacgccgacatgtatgatgatgtgttcgatgtctgaagga |
| wsnp_CA P12_c49 24_2241 879 QTL W SEQ ID NO.2 | 6A | TAGTTTTCTTCTTGGTTGCCTGTAATAACATCATAGGTTGAGGTTACCAGCCGCTCCGC ATAAAGCTTGAAAGTTGAACTTGCTGAAGCGGCGAGCCTCG[C/T]GAGCACCACCAC CACCGACAAGTATCAGGAGCACCTTTCCCTCCAGTACATGTAAATAAAAAGGAGTAT ACATATTTGTCCTATGTATCGATTTGAGAAT |
| BS00011 202_51 QTL B SEQ ID NO.3 | 6A | AACTTTATGCATGCGCCATTGCAGATCAATATCTAGTAGATCAATCAGCT[G/T]ctctcg cctaacaggatcaactccatcaactccaaatatcatgtgctcag |
| ORF256 SEQ ID NO.4 | MT | CACTAGCAGGTTTACTGCTTTCTATTTGCACTTTTGTATTAAGTTTCCTTATATATACGA TTTTTTATTATTTTCTATTTGTCTATTTTTCTTTTTAGTGCGTTTTATTTCGATTATTCTTC TCCCAATTTGCAATCTTTTCGGAGCCTCCTTCATTATTACTCTTCCTCCAGAGATTCAGG ATCCCCAAGCTCTAGCTCATTTAGCAGGGCTAAACTTCTATCTGAGCCTTTACGAGCA GGATCCTGGATGGGTTACGTTCATTCAGAACGAGCTTAATCACAATACCCCTCTGGAG GACATACCTGGACGGCTTAAGCTCTTCCTAATGGAAGAAAAGCTGTCTAGTATGCGA CAAGATGTCATTCAGGAATTTGTGGCGCTTTATCAAAGAATAGGGCCTTATCTACCGA TCGAGCCCTACTTGGTCGATGAAGCGCTTCGTTCCTATCTGGACCATATTCACGCAAC TGATTCTTTCACTGTTCTCCAAGCGTCTTATCAAGATCTGCGGGAGAATGAGGGAGG ATCTGTTTTCTTTAGAGATGCTGTTTCCCACAACCGGGATCTCCTTGAGGCGGAAAGC TCCGCAAGGAGGTGCCTGGAAGTGGAACAGAGGATCCGATGGGAAGAAATCCCCAA GAGCAAGGCAAGTCTCGAAAGAGCTGAGCACGAGCATGCTCTCGACTTGTTTAAGTC GGAGGATCTTAGAAGGGAATTAGAAAAAAAAAGAGCGGGGTAGCTCAGTAATTCTG ATTCTTTTCTCTTCCAGCCCCCGGGCGGGCCCCTCTTTCTTTTCTCTTCCAGCCCCCCGG CCCCTCTTTGATAAGGAAAGTTTGCATTTCTCAAATAAAAAATGACAAATATGGTTCG ATGGCTCTTCTC |
| TaMT_C MS SEQ ID NO.5 | MT | TATGYGACAAGATGTCATTCAGGAATTTGTGGCGCTTTATCAAAGA[A/G]TAGGGCCT TATCTACCGATCGAGCCCTACTTGGTCRATGAAGC |
| TaMT_C MS_part SEQ ID NO.6 | MT | TATGYGACAAGATGTCATTCAGGAATTTGTGGCGCTTTATCAAAGA[A/G]TAGSGCCT TATCTACYGATCGAGCCCTACTTGGTCRATGAAGC |
| HG67030 6.1 7019568 94-7019560 64 SEQ ID NO.7 | 3B | CACTACCCGCTTTACTACTTTCTATTTTTCTTATGTATTAAGTTTCCCTTAATATATAAT TTGATATTACTTTCAGTTTGTATATTTTATTCCTTTGTCGTTTTATATTATGCCTAATAC CAATTTTCAATCTTTTTAGTTCGTGCTTCTCGGCTCCTCCTTTCTGATCACTCTCCAGCC GGAGATTCAAGACCCCTAGGCTCTAGCTCATTTAGCAGGGCTAAACTTCTATCTGAGT CTTTACGAGCAGGATCCGAGATGGGTTACATTCATTCAGAACGAGCTTAATCACAATA CCCCCCAGAAGACATACCTGGGCGGCTTAAGCTCTTCCTAATGGAAGAAAAGCTCTCT TCTATGCGACAAGATGTCATTCAGGAATTTGTGGCGCTTTATCAAAGAGTAGCGCCTT ATCTACTGATCGAGCCCTACTTGGTCGATGAAGTGCTTCGTTCCTATCTGGACCATATT CACGCAACTTATTCTTTCACTGTTCTCTAAGCGTCTTATCAAGATCTGCGGGAGAATG AGGGAGGATCCGTTTTCTTTTGAAATGTTGTTTCCCACAACCGGGATCTCCTTGAGGC GGAAAGCTCCGCAAGGAGGTGCCTGGAAGTGGAACAGAGGATCCGGTGGGAAGAA ATCCCCAAGAGCAAGGCAAGTCTTGAAAGAGCTAAGCACGAGCATGCTCTCGACTTG TTTAAGTCGGAGAATCTTAGAAGGGAATTAGAAAAAAAAGCAGGGTAGCTCAGTAA TTCTGATTCTTTTCTCTTCCAGCCCCCTGGCCCCTCTTTCTTTTCTCTTCCAGCCCCCGG |
| | | GCGGGCCCCTCTTT |
| HG67030 6.1 5570443 10-5570446 43 SEQ ID NO.8 | 3B | GTTACGTTCATTCAGAACGAGCTTAATCACAATACCCCTCTGGAGGACATACCTGGAC GGCTTAAGCTCTTCCTAATGAAAGAAAAGCTGTCTAGTATGTGACAAGATGTCATTCA GGAATTTGTGGCGCTTTATCAAAGAATAGGGCCTTATCTACCGATCGAGCCCTACTTG GTCAATGAAGCGCTTCGTTCCTATCTGGACCATATTCACGCAACTGATTCTTTCACTGT TCTCCAAGCGTCTTATCAAGATCTGCGGGAGAATAAGGGAGGATCTGTTTTCTTTAGA GATGTTGTTTCCCACAACCGGGATCTCCTTGAGGCGGAAAGCT |
| Allel_X SEQ ID NO.9 | MT | CGATCGGTAGATAAGGCCCTAT |
| Allel_Y SEQ ID NO.10 | MT | CGATCGGTAGATAAGGCCCTAC |
| Common Primer SEQ ID NO.11 | MT | CATTCAGGAATTTGTGGCGCTTTATCAAA |

Hierzu wird auch auf das beigefügte Sequenzprotokoll hingewiesen.

Die folgende Tabelle ist eine Übersicht der Marker gemäß der Offenbarung. Es wird auch die Verwendung der Marker gemäß der Offenbarung für eine Hybridsamenproduktion beschrieben.

**Tabelle 8. Marker und Markerkombinationen und deren Verwendbarkeit u.a. zur Fertilitätsbestimmung**

| **Allel-kombi- nation** | **Tdurum_contig5066 7_306** | **wsnp_CAP12_c4924_22 41879** | **BS00011202_51** | **TaMT_CMS** | **Phänotyp** | **Verwendung für Hybridproduktion** |
|---|---|---|---|---|---|---|
| 1 | Rf3/Rf3 | M1/M1 | M2/M2 | steril | 100% fertil | Restorer |
| 2 | rf3/Rf3 | M1/M1 | M2/M2 | steril | 100% fertil | nicht verwenden |
| 3 | rf3/rf3 | M1/M1 | M2/M2 | steril | 0% fertil | CMS |
| 4 | Rf3/Rf3 | m1/M1 | M2/M2 | steril | instabil fertil | nicht verwenden |
| 5 | rf3/Rf3 | m1/M1 | M2/M2 | steril | instabil fertil | nicht verwenden |
| 6 | rf3/rf3 | m1/M1 | M2/M2 | steril | 0% fertil | nicht verwenden |
| 7 | Rf3/Rf3 | m1/m1 | M2/M2 | steril | instabil fertil | nicht verwenden |
| 8 | Rf3/Rf3 | m1/m1 | m2/M2 | steril | instabil fertil | nicht verwenden |
| 9 | rf3/Rf3 | m1/m1 | m2/M2 | steril | instabil fertil | nicht verwenden |
| 10 | rf3/rf3 | m1/m1 | m2/M2 | steril | 0% fertil | nicht verwenden |
| 11 | Rf3/Rf3 | m1/m1 | m2/m2 | steril | instabil fertil | nicht verwenden |
| 12 | rf3/Rf3 | m1/m1 | m2/m2 | steril | instabil fertil | nicht verwenden |
| 13 | rf3/rf3 | m1/m1 | m2/m2 | steril | 0% fertil | nicht verwenden |
| 14 | Rf3/Rf3 | M1/M1 | m2/M2 | steril | instabil fertil | nicht verwenden |
| 15 | rf3/Rf3 | M1/M1 | m2/M2 | steril | instabil fertil | nicht verwenden |
| 16 | rf3/rf3 | M1/M1 | m2/M2 | steril | 0% fertil | nicht verwenden |
| 17 | Rf3/Rf3 | M1/M1 | m2/m2 | steril | instabil fertil | nicht verwenden |
| 18 | rf3/Rf3 | M1/M1 | m2/m2 | steril | instabil fertil | nicht verwenden |
| 19 | rf3/rf3 | M1/M1 | m2/m2 | steril | 0% fertil | nicht verwenden |
| 20 | Rf3/Rf3 | m1/M1 | m2/M2 | steril | instabil fertil | nicht verwenden |
| 21 | rf3/Rf3 | m1/M1 | m2/M2 | steril | instabil fertil | nicht verwenden |
| 22 | rf3/rf3 | m1/M1 | m2/M2 | steril | 0% fertil | nicht verwenden |
| 23 | Rf3/Rf3 | m1/M1 | m2/m2 | steril | instabil fertil | nicht verwenden |
| 24 | rf3/Rf3 | m1/M1 | m2/m2 | steril | instabil fertil | nicht verwenden |
| 25 | rf3/rf3 | m1/M1 | m2/m2 | steril | 0% fertil | nicht verwenden |
| 26 | Rf3/Rf3 | M1/M1 | M2/M2 | fertil | 100% fertil | nicht verwenden |
| 27 | rf3/Rf3 | M1/M1 | M2/M2 | fertil | 100% fertil | nicht verwenden |
| 28 | rf3/rf3 | M1/M1 | M2/M2 | fertil | 100% fertil | Maintainer |
| 29 | Rf3/Rf3 | m1/M1 | M2/M2 | fertil | 100% fertil | nicht verwenden |
| 30 | rf3/Rf3 | m1/M1 | M2/M2 | fertil | 100% fertil | nicht verwenden |
| 31 | rf3/rf3 | m1/M1 | M2/M2 | fertil | 100% fertil | nicht verwenden |
| 32 | Rf3/Rf3 | m1/m1 | M2/M2 | fertil | 100% fertil | nicht verwenden |
| 33 | Rf3/Rf3 | m1/m1 | m2/M2 | fertil | 100% fertil | nicht verwenden |
| 34 | rf3/Rf3 | m1/m1 | m2/M2 | fertil | 100% fertil | nicht verwenden |
| 35 | rf3/rf3 | m1/m1 | m2/M2 | fertil | 100% fertil | nicht verwenden |
| 36 | Rf3/Rf3 | m1/m1 | m2/m2 | fertil | 100% fertil | nicht verwenden |
| 37 | rf3/Rf3 | m1/m1 | m2/m2 | fertil | 100% fertil | nicht verwenden |
| 38 | rf3/rf3 | m1/m1 | m2/m2 | fertil | 100% fertil | nicht verwenden |
| 39 | Rf3/Rf3 | M1/M1 | m2/M2 | fertil | 100% fertil | nicht verwenden |
| 40 | rf3/Rf3 | M1/M1 | m2/M2 | fertil | 100% fertil | nicht verwenden |
| 41 | rf3/rf3 | M1/M1 | m2/M2 | fertil | 100% fertil | nicht verwenden |
| 42 | Rf3/Rf3 | M1/M1 | m2/m2 | fertil | 100% fertil | nicht verwenden |
| 43 | rf3/Rf3 | M1/M1 | m2/m2 | fertil | 100% fertil | nicht verwenden |
| 44 | rf3/rf3 | M1/M1 | m2/m2 | fertil | 100% fertil | nicht verwenden |
| 45 | Rf3/Rf3 | m1/M1 | m2/M2 | fertil | 100% fertil | nicht verwenden |
| 46 | rf3/Rf3 | m1/M1 | m2/M2 | fertil | 100% fertil | nicht verwenden |
| 47 | rf3/rf3 | m1/M1 | m2/M2 | fertil | 100% fertil | nicht verwenden |
| 48 | Rf3/Rf3 | m1/M1 | m2/m2 | fertil | 100% fertil | nicht verwenden |
| 49 | rf3/Rf3 | m1/M1 | m2/m2 | fertil | 100% fertil | nicht verwenden |
| 50 | rf3/rf3 | m1/M1 | m2/m2 | fertil | 100% fertil | nicht verwenden |
| | | | | | | |
| | A=Rf3 | C=m1 | G=m2 | T. timopheevii = steril | | |
| | G=rf3 | T=M1 | T=M2 | T. aestivum = fertil | | |

Der Einsatz des Markers TaMT_CMS ermöglicht die Unterscheidung von T. timopheevii zu T. aestivum Zytoplasma. Wird das T. aestivum Zytoplasma detektiert, ist das Rf3 Gen auf Chromosom 1B nicht für die Fertilitätswiederherstellung relevant. Erfolgt die Detektion von T. timopheevii Zytoplasma wird die Information über das Vorhandensein von Rf3 relevant um eindeutig determinieren zu können, ob eine Fertilitätswiederherstellung erwartet werden kann (Allel: RF3/RF3 oder rf3/RF3) oder nicht (Allel: rf3/rf3). Dafür muss zusätzlich zu dem TaMT_CMS Marker (CMS Marker) der Tdurum_contig50667_306 Marker (Rf3 Marker) eingesetzt werden.

Die Kombination aus dem CMS und Rf3 Marker ermöglicht die Einteilung des Materials in R-, B- und A-Linien, die für die Erstellung einer Hybride die Materialkomponenten bilden (Tabelle 8). Der Kornertrag einer Weizen-Hybride ist davon abhängig wie die Ährenblüten bestäubt werden. Da der Weizen sich selbst bestäubt, ist es wichtig, dass jede Ährchenblüte für die Bestäubung Pollen produziert. Ist das nicht der Fall, kann die Effizienz des Kornansatzes unregelmäßig erfolgen. Um dem entgegen zu wirken, wurde eine Marker- und Allelkombination determiniert, die in einer Hybride mit T. timopheevii Zytoplasma eine vollständige Fertilität hervorbringen kann (Tabelle 8, Allelkombination 2). Dafür müssen die Hybride-Elternkomponenten für die R-Linie die Allelkombination 1, für die A-Linie Allelkombination 3 und für die B-Linie die Allelkombination 31 in der Tabelle 8 aufweisen. Nur die dominante Allelkombination aus den modifizierenden Markern wsnp_CAP12_c4924_2241879 und BS00011202_51 in der Hybride kann die Fertilitätswiederherstellung und damit einhergehende vollständige Ähreneinkörnung gewährleisten.

Je geringer die Anzahl der dominanten modifizierenden Marker in der Hybride enthalten sind, desto instabiler werden die Fertilitätswiederherstellung und somit die Ertragsausbeute.

Eine Hybride, die von sich aus eine vollständige Fertilitätswiederherstellung bewerkstelligen kann, benötigt keine Restorer Linien als zusätzliche Bestäuber. Dies würde zum einen die mögliche Inhomogenität im Feld verhindern zum anderen Ertragseinbußen minimieren.

Der Einsatz des CMS Markers und/oder in Kombination mit den anderen hier beschriebenen Markern kann für offizielle Sortenüberprüfungen zum Nachweis von T. timopheevii Zytoplasma herangezogen werden.

### Referenzen

Benke et al. 2014. The genetic basis of natural variation for iron homeostasis in the maize IBM population.
Barkan et al. 2012. A Combinatorial Amino Acid Code for RNA Recognition by Pentatricopeptide Repeat Proteins.
Uyttewaal et al. 2008. Characterization of Raphanus sativus Pentatricopeptide Repeat Proteins Encoded by the Fertility Restorer Locus for Ogura Cytoplasmic Male Sterility.
Ahmed et al. 2001. QTL analysis of fertility-restoration against cytoplasmic male sterility in wheat.
Kazama et al., 2008. Suppression mechanism of mitochondrial ORF79 accumulation by Rf1 protein in BT-type cytoplasmic male sterile rice.
Liu et al., 2016. A Mitochondria-Targeted PPR Protein Restores pol Cytoplasmic Male Sterility by Reducing orf224 Transcript Levels in Oilseed Rape
Whitford et al. 2013. Hybrid breeding in wheat: Technologies to improve hybrid wheat seed production
De Givry et al., 2004. Carhta Gene: multipopulation integrated genetic and radiation hybrid mapping.
Rongxia et al., 2002. Analysis of the fertility restoring gene Rf6 in T. timopheevii cytoplasmic male sterility of wheat with ISSR markers
El-Shehawi et al. 2012. Genetic Fingerprinting of Wheat and Its Progenitors by Mitochondrial Gene orf256.
Becker 1993. Buch: Pflanzenzüchtung.

## Patentansprüche

1. CMS Markergen charakterisiert durch die Sequenz SEQ ID NO.5, ein Fragment davon mit mindestens 12 Basenpaaren (bp) oder mit 12 bis 30 bp, oder eine Primersequenz aufweisend mindestens 12 Basenpaaren (bp) oder aufweisend 12 bis 30 bp , der Sequenz SEQ ID NO. 5, vorzugsweise SEQ ID NO.9, 10, und 11, die aufweist drei Polymorphismen **gekennzeichnet durch** zwei R- (A/G) und einen Y-Polymorphismus (C/T) und gegebenenfalls einen S-Polymorphismus (G/C) und/oder ein Y-Polymorphismus (C/T)

2. CMS Markergen charakterisiert durch die Sequenz SEQ ID NO.5, ein Fragment davon mit mindestens 12 Basenpaaren (bp) oder mit 12 bis 30 bp, oder eine Primersequenz aufweisend mindestens 12 Basenpaaren (bp) oder aufweisend 12 bis 30 bp, der Sequenz SEQ ID NO.5, vorzugsweise SEQ ID NO.9, 10, und 11, die aufweist drei Polymorphismen **gekennzeichnet durch** zwei R- (A/G) und einen Y-Polymorphismus (C/T) und gegebenenfalls einen S-Polymorphismus (G/C) und/oder ein Y-Polymorphismus (C/T) zur Verwendung in einem Verfahren zur Bestimmung des Zytoplasmas einer Weizenpflanze, Teilen einer Weizenpflanze oder/und einer Weizenpflanzenzelle, vorzugsweise wobei die Weizenpflanze, Teilen einer Weizenpflanze oder/und die Weizenpflanzenzelle ein T. timopheevii oder ein T. aestivum ist.

3. Primersequenz geeignet zum Nachweis einer Sequenz in einer Weizenzelle (Triticae spec.), **gekennzeichnet durch** mindestens 12 Basenpaaren (bp) oder durch 12 bis 30 bp aus der Sequenz SEQ ID NO.5, vorzugsweise SEQ ID NO.9, 10, und 11, und die aufweist zwei R-Polymorphismen (A/G) und einen Y-Polymorphismus (C/T) und gegebenenfalls einen S-Polymorphismus (G/C) und/oder ein Y-Polymorphismus (C/T)

4. Verfahren zur Bestimmung des Zytoplasmas einer Weizenpflanze, Teilen einer Weizenpflanze oder/und einer Weizenpflanzenzelle, vorzugsweise wobei die Weizenpflanze, Teilen einer Weizenpflanze oder/und die Weizenpflanzenzelle ein T. timopheevii oder ein T. aestivum ist, wobei mindestens eine Primersequenz gemäß Anspruch 3 verwendet wird.

5. Kit umfassend mindestens eine Primersequenz gemäß Anspruch 3 und gegebenenfalls eine Polymerase, Nucleotide und Puffer.

6. Schnelltest zum Nachweis von Zytoplasma in einer Probe, umfassend die Schritte Verwendung von Komponenten umfassend mindestens CMS Markergen charakterisiert durch die Sequenz SEQ ID NO.5, ein Fragment davon mit mindestens 12 Basenpaaren (bp) oder mit 12 bis 30 bp, oder eine Primersequenz aufweisend mindestens 12 Basenpaaren (bp) oder aufweisend 12 bis 30 bp , der Sequenz SEQ ID NO.5, vorzugsweise SEQ ID NO.9, 10, oder 11, Temperieren, vorzugsweise Aufheizen der Komponenten bis zu einer Aufschmelztemperatur, Temperieren, vorzugsweise Abkühlen bis auf eine Hybridisierungstemperatur, und Durchführen einer Visualisierungsreaktion zur Sichtbarmachung des Vorhandensein von Zytoplasma von T. timopheevii oder von T. aestivum.

7. Ein oder mehrere Markergene oder eine Kombination davon, vorzugsweise mindestens 2 oder 3 Markergene, wobei das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm in Kombination mit einem CMS Markergen nach Anspruch 1 oder einer Primersequenz nach Anspruch 3 zur Verwendung in einem Verfahren zur Bestimmung der Fertilität oder/und der Fertilitätswiederherstellung oder/und von Restorergenen oder/und von ausgewählten Allelen oder einer Allelkombination in einer Weizenpflanze oder Teilen davon oder in einer Weizenzelle.

8. Markergen oder Kombination von Markergenen nach Anspruch 7, wobei der oder die Markergene ausgewählt sind aus der Gruppe bestehend aus den Markern QTL T, QTL B und QTL W.

9. Verfahren zum Nachweis der Fertilität oder/und der Fertilitätswiederherstellung oder/und von Restorergenen in einer Weizenpflanze oder Teilen einer Weizenpflanze oder einer Weizenzelle, wobei ein genetischer Marker oder eine Kombination von genetischen Markern verwendet wird ausgewählt aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm, das Markergen oder die Markergene vorzugsweise ausgewählt werden aus der Gruppe bestehend aus QTL T, QTL B und QTL W, und die Fertilität oder der Grad der Fertilität bestimmt wird in Kombination mit einem CMS Markergen nach Anspruch 1 oder einer Primersequenz nach Anspruch 3.

10. Verfahren zum Nachweis eines ausgewählten Allels oder/und einer ausgewählten Allelkombination in einer Weizenpflanze oder Teilen einer Weizenpflanze, wobei ein genetischer Marker oder eine Kombination von genetischen Markern ausgewählt aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm oder ausgewählt aus der Gruppe QTL T, QTL B und QTL W, verwendet werden, in Kombination mit einem CMS Markergen nach Anspruch 1 oder einer Primersequenz nach Anspruch 3 und die ausgewählten Allele bestimmt werden.

11. Verfahren zur Selektion einer Pflanze, die ein oder mehrere funktionelle Restorergene für eine zytoplasmatische männliche Sterilität (CMS) in Weizen aufweist, umfassend die Schritte:
a. Screenen einer Pflanzenpopulation oder einer Sammlung oder Gruppe von Pflanzen für mindestens einen oder zwei Marker in Kombination mit einem CMS Markergen nach Anspruch 1 oder einer Primersequenz nach Anspruch 3, vorzugsweise drei Marker, wobei die Markernukleinsäure ein Allel umfasst, das einen Allellink (LD /linkage disequilibrium) mit einem oder mehreren Restorergenen darstellt oder sich in einem oder mehreren Restorergenen befindet,
b. Nachweisen der Markernukleinsäuren,
c. Identifizieren der Pflanzen, die die Markernukleinsäuren aufweisen,
d. Selektion der Pflanzen, die die Markernukleinsäuren aufweisen,
vorzugsweise wobei das Verfahren die zusätzlichen Schritte umfasst a. Erhalten der Pflanze, die ein oder mehrere Restorergene enthält, b. Kreuzen der Pflanze mit einer zweiten Pflanze, die ein oder mehrere Restorergene enthält, um Nachkommenpflanzen zu erhalten, c. Screenen der Nachkommenpflanzen für zwei oder drei der Marker aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm oder für zwei oder drei der Marker QTL T, QTL B und QTL W, d. Selektion der Pflanzen, die eine oder mehrere Markernukleinsäuren ausgewählt von QTL T, QTL B und QTL W aufweist,
vorzugsweise wobei die Schritte a.) bis d.) wiederholt werden, um eine Pflanze zu erhalten, die alle drei der Marker aus der Gruppe, bei der das Markergen mit einem oder mehreren Restorergenen assoziiert ist (Vertrauensintervall) innerhalb von 17 Mb bzw. 50 cM auf Chromosom 1B oberer Arm, innerhalb von 5 Mb bzw. 26 cM auf Chromosom 6A unterer Arm oder/und innerhalb von 1 Mb bzw. 7 cM auf Chromosom 6A oberer Arm aufweist oder die alle drei der Marker QTL T, QTL B und QTL W aufweist (Pyramidisierung).

12. Verwendung von mindestens zwei genetischen Markern nach Anspruch 1, 7 oder 8 zum Screenen oder zur Selektion von Pflanzen, oder Pflanzenzellen oder/und zur Pyramidisierung oder Verwendung mindestens einem Primer, vorzugsweise drei Primern nach Anspruch 3 zum Screenen oder zur Selektion von Pflanzen oder Pflanzenzellen.

13. Marker-Kombination nach Anspruch 7 oder 8, oder Verfahren nach einem der Ansprüche 9 bis 11, wobei die Kombination die Marker QTL T und QTL B, oder QTL T und QTL W umfasst oder aus ihr besteht bzw. das Verfahren eine Kombination der Marker QTL T und QTL B, oder der Marker QTL T und QTL W oder der Marker QTL T, QTL B und QTL W verwendet.

14. Marker-Kombination nach Anspruch 7 oder 8 oder Verfahren nach Anspruch 9 bis 11, wobei die Marker auf Chromosom 1B oder/und mitochondrialer DNS liegen.

15. Verfahren zur Bestimmung eines Restorers, eines Maintainers oder/und einer CMS, wobei ein Markergen gemäß Anspruch 1 (CMS) und ein oder zwei Markergene gemäß Anspruch 9 oder/und 10 verwendet werden.
